Europäisches Patentamt

European Patent Office

Office européen des brevets

(11) Veröffentlichungsnummer: **0 456 086 B1**

## EUROPÄISCHE PATENTSCHRIFT

(45) Veröffentlichungstag der Patentschrift: **02.08.95**

(51) Int. Cl.⁶: **C12N 9/22**

(21) Anmeldenummer: **91106981.3**

(22) Anmeldetag: **30.04.91**

(54) **TYP II-Restriktionsendonuklease SwaI.**

(30) Priorität: **07.05.90 DE 4014524**

(43) Veröffentlichungstag der Anmeldung:
**13.11.91 Patentblatt 91/46**

(45) Bekanntmachung des Hinweises auf die
Patenterteilung:
**02.08.95 Patentblatt 95/31**

(84) Benannte Vertragsstaaten:
**AT BE CH DE DK ES FR GB GR IT LI LU NL SE**

(56) Entgegenhaltungen:

NUCLEIC ACIDS RESEARCH - SEOUENCES
SUPPLEMENT Band 18, 25. April 1990, Seiten
2331-2365, Eynsham, Oxford, GB; R.J. RO-
BERTS: "Restriction enzymes and their isoschizomers"

(BOEHRINGER MANNHEIM GMBH)

DATABASE WPIL/DERWENT Zusammenfassung Nr. 90-097738(13), 1990, Derwent Publications Ltd., London, GB; & SU - A- 1486512
(BIOL. ACTIVE MAT. RES. et al.) 15.06.1989

(73) Patentinhaber: **BOEHRINGER MANNHEIM GMBH**

**D-68298 Mannheim (DE)**

(72) Erfinder: **Prinz, Barbara, Dr.**
**Wasenöschstrasse 15/1**
**W-7990 Friedrichshafen (DE)**
Erfinder: **Lechner, Max, Dr.rer.nat.**
**Fischhaberstrasse 61**
**W-8122 Penzberg (DE)**
Erfinder: **Frey, Bruno, Dr.rer.nat.**
**Hochfeldstrasse 50**
**W-8122 Penzberg (DE)**
Erfinder: **Jarsch, Michael, Dr.rer.nat.**
**Unterkarpfsee 11**
**W-8173 Bad Heilbrunn (DE)**

FEBS LETTERS Band 143, Nr. 2, Juli 1982, Seiten 296-300, Amsterdam, NL; P.R. WHITE-HEAD et al.: "AhaIII: A restriction endonuclease with a recognition sequence containing only A:T basepairs"

NUCLEIC ACIDS RESEARCH Band 11, Nr. 16, 1983, Seiten 5467-5474, Oxford, GB; I.J. PURVIS et al.: "Isolation and characterisation of DraI, a type II restriction endonuclease recognising a sequence containing only A:T basepairs, and inhibition of its activity by uv irradiation of substrate DNA"

**Beschreibung**

Die Erfindung betrifft die neue Typ II-Restriktionsendonuklease SwaI, ein Verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolysiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch besteht immer noch Bedarf an der Schaffung weiterer Typ II-Restriktionsendonukleasen, die für DNA-Sequenzen spezifisch sind und die bisher von keiner der bekannten Restriktionsendonukleasen erkannt werden. Der Erfindung liegt daher die Aufgabe zu Grunde, eine neue Restriktionsendonuklease zur Verfügung zu stellen, welche eine bisher von keinem derartigen Enzym erkannte Sequenz spezifisch zu erkennen und zu spalten vermag.

Gelöst wird diese Aufgabe erfindungsgemäß durch eine Typ-II-Restriktionsendonuklease mit der Erkennungssequenz und der durch die Markierung definierten Spaltstelle

$$5'\text{-ATTT} \mid \text{AAAT-}3'$$
$$3'\text{-TAAA} \mid \text{TTTA-}5' \ ,$$

welche aus Mikroorganismen der Gattung staphylococcus erhältlich ist.

Die erfindungsgemäße neue Restriktionsendonuklease, die im folgenden als SwaI bezeichnet wird, hat ein Temperaturoptimum bei 25°C. Das Enzym besitzt eine gute Aktivität zwischen pH 7,0 und pH 8,0 in 50 mmol/l Tris/HCl-Puffer mit 1,0 mmol/l DTE (Dithiothreitol), 10 mmol/l MgCl und 100 mmol/l NaCl. Das pH-Optimum liegt bei pH 7,5.

Die Erkennungssequenz läßt sich durch vollständigen Verdau der DNAs der Viren SV40 und Adeno 2, der Phagen Lambda, T7 und phiX174 und dem Phagenderivat M13mp7 sowie den Plasmiden pBR 322 und pBR 328 bestätigen. Diese DNA-Moleküle werden mit SwaI behandelt.

Tabelle 1 zeigt einen Vergleich der experimentell beobachteten Schnittstellenspezifität mit einer computerermittelten Schnittstellenspezifität für ein Enzym, welches folgende Sequenz erkennt:
5'-ATTTAAAT-3'

Tabelle I

| DNA | Anzahl der experimentell bestimmten Schnittstellen | Anzahl der durch Computeranalyse bestimmte Schnittstellen | Experimentell bestimmte Fragmentlängen (Basenpaare) | Durch Computeranalyse bestimmte Fragmentlängen (Basenpaare) | Spaltpositionen ermittelt durch Computeranalyse (bei Basenpaar) |
|---|---|---|---|---|---|
| SV40 | 1 | 1 | 5250 | 5243 | 1797 |
| M13mp7 | 1 | 1 | 7200 | 7229 | 6761 |
| Phix174 | 0 | 0 | 0 | 0 | 0 |
| PBR322 | 0 | 0 | 0 | 0 | 0 |
| pBR328 | 0 | 0 | 0 | 0 | 0 |
| Lambda | 0 | 0 | 0 | 0 | 0 |
| T7 | 1 | 1 | 34000 / 6000 | 33966 / 5970 | 5970 |
| Ad2 | 1 | 1 | 28900 / 7000 | 28904 / 7033 | 28904 |

Die Spaltposition innerhalb der Erkennungssequenz des Enzyms läßt sich an einem M13-Derivat, das im Abstand von ca. 30 - 200 Basen zur Bindungsstelle des universalen Sequenzierprimers (Messing, J. et al. (1981) Nucl. Acids Res. 9, 309 - 321) diese Erkennungssequenz trägt, bestimmen. An einzelsträngiger DNA des M13-Derivates werden zunächst mit dem universalen Sequenzierprimer Sequenzreaktionen nach der Didesoxy-Kettenabbruchmethode durchgeführt (Sanger, F., et al. (1977) Proc. Natl. Acad. Sci. USA 74, 560 - 564, Messing, J. et al. (1981) Nucl. Acids Res. 9, 309 - 321).

Parallel dazu wird der Sequenzierprimer mit T4-Polynukleotid-Kinase und [$\gamma$-$^{32}$P]ATP am 5'-Ende radioaktiv markiert. Nach Anhybridisieren dieses 5'-endmarkierten Sequenzierprimers an die einzelsträngige

4

M13-DNA wird in einer Auffüllreaktion mit DNA Polymerase I, (Klenow Enzym) und einer Desoxynukleotidtriphosphatmischung aus dATP, dCTP, dGTP und dTTP eine partiell doppelsträngige DNA hergestellt. Diese DNA, deren neusynthetisierter Strang am 5'-Ende radioaktiv markiert ist, wird mit der Restriktionsendonuklease SgrAI gespalten. Die Hälfte des Spaltansatzes wird zusätzlich noch mit T4 DNA Polymerase in Gegenwart einer Mischung aller vier Desoxynukleotidtriphosphate behandelt, um glatte DNA-Enden zu erhalten.

Die Analyse der Reaktionsprodukte erfolgt durch Elektrophorese auf Sequenziergelen (8 mol/l Harnstoff, 5 % Polyacrylamid) und anschließender Autoradiographie. Die Interpretation der Ergebnisse wird nach Brown, N.L. und Smith, M. (Methods in Enzymology 65 (1980) 391 - 401) durchgeführt. Durch Vergleich der Laufstrecken der radioaktiv markierten Fragmente mit der Sequenzleiter wird die Lage der Spaltstelle bestimmt. Die zusätzlich mit T4 DNA Polymerase behandelten Proben Zeigen im Vergleich zu der lediglich mit SwaI gespaltenen Probe eine identische Laufstrecke der Banden. Damit ist gezeigt, daß SwaI ein glattes DNA-Ende erzeugt. Die Spaltung von SwaI erfolgt innerhalb der Erkennungssequenz daher mit folgender Spezifität:

$$5'-\text{ATTT} \mid \text{AAAT}-3'$$
$$3'-\text{TAAA} \mid \text{TTTA}-5'$$

Die experimentell bestimmte Anzahl der Schnittstellen ist identisch mit der Anzahl der Schnittstellen, die durch Computeranalyse mit den verschiedenen DNAs für die Sequenz

5'-ATTTAAAT-3'

erhalten wurde (Tabelle I). Zusätzlich wurden diese Daten noch mit den Tabellen aus Gene 10 (1980) 357 - 370, verglichen.

Vorzugsweise wird SwaI gewonnen, indem man Mikroorganismen der Gattung Staphylococcus vorzugsweise der Art Staphylococcus warneri züchtet und das Enzym aus den Zellen gewinnt. Besonders bevorzugt wird Staphylococcus warneri DSM 5872 verwendet.

Der Mikroorganismus Staphylococcus warneri ist bei der Deutschen Sammlung von Mikroorganismen (DSM), Mascheroder Weg 1b, 3300 Braunschweig, BRD, hinterlegt und trägt die Hinterlegungsnummer DSM 5872.

Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Adeno2-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Die für die Gewinnung des Enzyms verwendeten Mikroorganismen wachsen aerob in Brain Heart Infusion Medium der Firma Difco. Die optimalen Wachstumsbedingungen sind bei 37 °C, pH 6,5 - 7,5. Die Verdoppelungszeit beträgt etwa 3 Stunden.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen über eine French-Presse aufgeschlossen. Die weitere Reinigung des Überstands wird vorzugsweise über Affinitätschromatographie, und Ionentauscherchromatographie durchgeführt. Als Material für die Affinitätschromatographie geeignet ist beispielsweise Heparin-Sepharose CL-6B (Pharmacia). Ein geeigneter Kationenaustauscher ist beispielsweise Phospho-Cellulose (Whatman).

Als Anionentauscher geeignet ist das unter dem Namen DEAE fast-flow (Pharmacia) erhältliche Produkt. Aber auch andere Chromatographiematerialien, die dem Fachmann bekannt sind, sind geeignet.

Die folgenden Beispiele erläutern die Erfindung weiter.

Beispiel 1

Staphylococcus warneri DSM 5872 wird bei 37 °C 12-15 Stunden gezüchtet und am Ende der logarithmischen Phase geerntet. Als Kulturmedium wird Brain Heart Medium (Difco) verwendet.

Die Zellpaste (30 g Naßgewicht) wird in 2,4 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.000 lb/inch$^2$ aufgeschlossen und der Niederschlag abgetrennt. Der überstand wird mit NH$_4$Cl versetzt (Endkonzentration 0,1 mol/l). Durch eine Polymin-Fällung werden die Nucleinsäuren entfernt. Anschließend wird der abzentrifugierte Überstand auf einer Heparin-Sepharose-Säule fraktioniert. Zur Elution wird ein Gradient von 0 - 1 mol/l NaCl verwendet. SwaI wird in den Fraktionen zwischen 0,4 und 0,6 mol/l NaCl gefunden. Die aktiven Fraktionen werden gegen Puffer B (40 mmol/l Tris-HCl, pH 8,0; 0,1 mmol/l EDTA; 7 mmol/l 2-Mercaptoethanol; 10% (w/v) Glycerin) equilibriert und auf einer DEAE-fast-flow-Säule fraktioniert. Zur Elution wird ein Gradient von 0 - 0,5 mol/l NaCl verwendet. Die aktiven Fraktionen werden gegen Puffer B dialysiert.

Anschließend werden sie auf eine Phospho-Cellulose-Säule, welche mit Puffer B equilibriert wurde, aufgegeben. Zur Elution wird ein Gradient von 0 - 1 mol/l NaCl in Puffer B verwendet. SwaI wird in den Fraktionen zwischen 0,3 und 0,5 mol/l NaCl gefunden.

Die aktiven Fraktionen werden zusammengegeben und gegen Lagerpuffer (20 mmol/l Tris-HCl, pH 8,0, 10 mmol/l 2-Mercaptoethanol, 100 mmol/l NaCl, 0,1 mmol/l EDTA und 50% (v/v) Glycerin) dialysiert.

Beispiel 2

Bestimmung der Aktivität

Definition der Enzymeinheiten: 1 U SwaI spaltet 1 $\mu$g Adeno2-DNA innerhalb 1 Stunde bei 25°C in 25 $\mu$l Endvolumen.

Zu einer Mischung von 2,5 $\mu$l Inkubationspuffer (500 mmol/l Tris-HCl, pH 7,5, 37°C, 100 mmol/l Magnesiumchlorid, 1 mol/l NaCl und 10 mmol/l DTE) werden 17,9 $\mu$l Wasser und 3,6 $\mu$l Adeno2 DNA (optische Dichte: 5,6 OD/ml) sowie 1 $\mu$l SwaI-Lösung (1 U/$\mu$l) zugegeben. Die Lösung wird eine Stunde bei 25°C inkubiert, auf Eis gekühlt und mit 5 $\mu$l eines Abstopp-Reagenses, bestehend aus 7 mmol/l Harnstoff, 20 % (w/v) Saccharose, 60 mmol/l EDTA und 0,01 % (w/v) Bromphenolblau versetzt. Anschließend wird eine Trennung durch Elektrophorese in 1 % Agarose-Gelen für 3 - 4 Stunden bei 100 V durchgeführt. Die erhaltenen Banden werden über Vergleich mit einem DNA-Längen-standard identifiziert.

**Patentansprüche**

1. Typ II-Restriktionsendonuklease mit der Erkennungssequenz und der durch die Markierung charakterisierten Schnittstelle

$$5'\text{-ATTT} \mid \text{AAAT-}3'$$
$$3'\text{-TAAA} \mid \text{TTTA-}5' \ ,$$

erhältlich aus Mikroorganismen der Gattung Staphylococcus.

2. Restriktionsendonuklease nach Anspruch 1, dadurch gekennzeichnet, daß sie aus Staphylococcus warneri DSM 5872 erhältlich ist.

3. Restriktionsendonuklease nach den Ansprüchen 1 oder 2, gekennzeichnet durch ein Temperatur-Optimum bei 25 °C und ein pH-Optimum bei 7,5.

4. Verfahren zur Gewinnung einer Typ II-Restriktionsendonuklease mit der Erkennungssequenz und der durch die Markierung charakterisierten Spaltstelle

$$5'\text{-ATTT} \mid \text{AAAT-}3'$$
$$3'\text{-TAAA} \mid \text{TTTA-}5' \ ,$$

dadurch gekennzeichnet, daß man Mikroorganismen der Gattung Staphylococcus züchtet und das Enzym aus den Zellen gewinnt.

5. Verfahren nach Anspruch 4, dadurch gekennzeichnet, daß man Staphylococcus warneri DSM 5872 züchtet.

6. Verfahren nach den Ansprüchen 4 und 5, dadurch gekennzeichnet, daß man die Zellen aufschließt und den Zellüberstand gewinnt.

7. Verfahren nach Anspruch 6, dadurch gekennzeichnet, daß man zur Feinreinigung den Zellüberstand einer Affinitätschromatographie, einer Anionenaustauschchromatographie und einer Kationenaustauschchromatographie unterwirft.

8. Verfahren nach Anspruch 7, dadurch gekennzeichnet, daß man zur Affinitätschromatographie trägerfixiertes Heparin verwendet.

9. Verwendung einer Typ II-Restriktionsendonuklease mit der Erkennungssequenz und der durch die Markierung charakterisierten Schnittstelle

$$5'\text{-ATTT} \mid \text{AAAT-}3'$$
$$3'\text{-TAAA} \mid \text{TTTA-}5' \ ,$$

erhältlich aus Mikroorganismen der Gattung Staphylococcus, zur Erkennung und Spaltung der komplementären doppelstänigigen DNA-Sequenz
    5'-ATTTAAAT-3'

**Claims**

1. Type II restriction endonuclease with the recognition sequence and the cleavage position characterised by the marking

$$5"\text{-ATTT} \mid \text{AAAT-}3'$$
$$3'\text{-TAAA} \mid \text{TTTA-}5'$$

obtainable from micro-organisms of the genus Staphylococcus.

2. Restriction endonuclease according to claim 1, characterised in that it is obtainable from Staphylococcus warneri DSM 5872.

3. Restriction endonuclease according to claims 1 or 2, characterised by a temperature optimum at 25°C and a pH optimum at 7.5.

4. Process for the obtaining of a type II restriction endonuclease with the recognition sequence and the cleavage position characterised by the marking

$$5'\text{-ATTT} \mid \text{AAAT-}3'$$
$$3'\text{-TAAA} \mid \text{TTTA-}5'$$

characterised in that one cultures micro-organisms of the genus Staphylococcus and obtains the enzyme from the cells.

5. Process according to claim 4, characterised in that one cultures Staphylococcus warneri DSM 5872.

6. Process according to claims 4 and 5, characterised in that one digests the cells and obtains the cell supernatant.

7. Process according to claim 6, characterised in that, for the high purification, one subjects the cell supernatant to an affinity chromatography, to an anion exchange chromatography and to a cation

EP 0 456 086 B1

exchange chromatography.

8.  Process according to claim 7, characterised in that one uses carrier-fixed heparin for the affinity chromatography.

9.  Use of a type II restriction endonuclease with the recognition sequence and the cleavage position characterised by the marking

$$5'-ATTT \mid AAAT-3'$$
$$3'-TAAA \mid TTTA-5'$$

obtainable from micro-organisms of the genus Staphylococcus for the recognition and cleavage of the complementary double-stranded DNA sequence
5'-ATTTAAAT-3'

**Revendications**

1.  Endonucléase de restriction de type II, avec la séquence de reconnaissance et le site de coupure caractérisé par le marqueur

$$5'-ATTT \mid AAAT-3'$$
$$3'-TAAA \mid TTTA-5',$$

qui peut être obtenue à partir de micro-organismes du genre Staphylococcus.

2.  Endonucléase de restriction selon la revendication 1, caractérisée en ce qu'elle peut être obtenue à partir de Staphylococcus warneri DSM 5872.

3.  Endonucléase de restriction selon les revendications 1 ou 2, caractérisée par une température optimale de 25°C et un pH optimal de 7,5.

4.  Procédé d'obtention d'une endonucléase de restriction de type II, avec la séquence de reconnaissance et le site de coupure caractérisé par le marqueur

$$5'-ATTT \mid AAAT-3'$$
$$3'-TAAA \mid TTTA-5',$$

caractérisé en ce que l'on cultive des micro-organismes du genre Staphylococcus et on récupère l'enzyme à partir des cellules.

5.  Procédé selon la revendication 4, caractérisé en ce que l'on cultive Staphylococcus warneri DSM 5872.

6.  Procédé selon l'une des revendications 4 et 5, caractérisé en ce que l'on effectue une rupture des cellules et on récupère le surnageant de cellules.

7.  Procédé selon la revendication 6, caractérisé en ce que pour la purification, on soumet le surnageant de cellules à une chromatographie d'affinité, une chromatographie d'échange d'anions ou une chromatographie d'échange de cations.

8.  Procédé selon la revendication 7, caractérisé en ce que pour la chromatographie d'affinité, on utilise de l'héparine fixée à un support.

8

**9.** Utilisation d'une endonucléase de restriction de type II, avec la séquence de reconnaissance et le site de coupure caractérisé par le marqueur

$$5'\text{-ATTT} \mid \text{AAAT-3'}$$
$$3'\text{-TAAA} \mid \text{TTTA-5'},$$

qui peut être obtenue à partir de micro-organismes du genre Staphylococcus, pour la reconnaissance et la coupure de la séquence d'ADN complémentaire double brin
5'-ATTTAAAT-3'0.